(19) Europäisches Patentamt
European Patent Office
Office européen des brevets

(11) **EP 3 389 526 B1**

(12) **EUROPEAN PATENT SPECIFICATION**

(45) Date of publication and mention
of the grant of the patent:
**05.08.2020 Bulletin 2020/32**

(51) Int Cl.:
***A61B 18/02*** *(2006.01)* ***A61B 34/10*** *(2016.01)*

(21) Application number: **16836090.7**

(22) Date of filing: **12.12.2016**

(86) International application number:
**PCT/IB2016/057541**

(87) International publication number:
**WO 2017/103762 (22.06.2017 Gazette 2017/25)**

(54) **A MACHINERY FOR SURGICAL INTERVENTIONS OF CRYOABLATION**

MULTIFUNKTIONSAPPARAT FÜR CHIRURGISCHE KRYOABLATIONSVERFAHREN

MACHINE POUR INTERVENTIONS CHIRURGICALES DE CRYOABLATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **14.12.2015 IT UB20159658**

(43) Date of publication of application:
**24.10.2018 Bulletin 2018/43**

(73) Proprietors:
• **Penco, Andrea**
**16129 Genova (IT)**
• **Avalle, Leopoldo**
**16167 Genova (IT)**
• **Bertelli, Alessandro Camillo**
**16167 Genova (GE) (IT)**
• **Vercelloni, Massimo Alessandro**
**20124 Milano (MI) (IT)**
• **Gulli, Vito Giampiero**
**16031 Pieve Ligure (GE) (IT)**

(72) Inventor: **AVALLE, Leopoldo**
**16147 Genova (IT)**

(74) Representative: **Emmi, Mario**
**Studio Brevetti Turini Srl**
**Viale Matteotti, 25**
**50121 Firenze (FI) (IT)**

(56) References cited:
**US-A1- 2002 040 220    US-A1- 2006 155 267**
**US-A1- 2008 033 419    US-A1- 2009 171 203**

• **Hugo Talbot ET AL: "Interactive Planning of
Cryotherapy Using Physically-Based Simulation
Interactive Planning of Cryotherapy Using
Physics-Based Simulation", , 1 February 2014
(2014-02-01), page 918200, XP055292622,
Retrieved from the Internet:
URL:https://hal.inria.fr/hal-00918200/file
/Talbot_H.pdf [retrieved on 2016-08-01] & Team
Mimesis: "Cryoablation simulation using SOFA",
YouTube, 24 November 2015 (2015-11-24), pages
1-4, XP054976701, Retrieved from the Internet:
URL:https://www.youtube.com/watch?v=VJNjgc
13ypE [retrieved on 2016-08-02]**

**Description**

Technical field

The present invention refers to the technical field of machineries for surgery and interventions of the cryogenic type.

In particular, the invention refers to an innovative machinery for allowing an operation, according to the cryogenic technique, with maximum precision and efficacy.

Prior art

The technique of the cryosurgery has been known and applied for a long time.

Its operation is based on the utilization of cold, that is particularly low temperature (approximately -100°C and over), in order to freeze tissues of a predetermined area, causing its death. In particular, cooling/heating cycles are foreseen and they are repeated for a predetermined period to cause the death of the cells hit by such cycle.

This kind of surgery is thus used to attack neoplasia, producing a cycle of repeated local decreases/increases of the temperature in pathological areas for causing the death of almost all cells and preventing them from reproducing and/or growing at the same time.

The machinery used in background art foresees a cooling system which is generally with open cycle. Probes are then foreseen for penetrating into the specific points of the tissue. Such probes (so-called cryodes) are cooled and remove heat locally, thus causing the freezing of the whole area hit by the advancement of the cold front. Other warm probes (also so-called sentinels) are foreseen for protecting adjacent healthy tissues from cryogenic effects of the cryodes.

Current machineries have the following various technical inconveniences.

In particular, over the prior art, there is no managing system which allows to determine precisely the number and the position of the suitable placement of the probes for optimizing the success of the intervention. In fact, the advancement of the cold front is function of the shape, position and size of the cooling apparatus. Over the prior art, the surgeon, basing on his experience, determines the number of probes to be used and arranges them in the most appropriate way for him. Such positioning cannot be precise and the risk is that, depending on cases, either the ill tissue is only in part hit by the cold front or the cold front advances damaging also healthy tissues. In that way, as per prior art, it is difficult to hit ill cells efficaciously while preserving the healthy tissue as much as possible.

Several documents are known dealing with potential simulation systems that allow the simulation of a cryoablative intervention.

For example, it is known the publication "Hugo Talbot, Myriam Lekkal, Remi Bessard-Duparc, Stephane Cotin. Interactive Planning of Cryotherapy Using Physically-Based Simulation. MMVR 21 - Medicine Meets Virtual Reality - 2014, Feb 2014, Manhattan Beach, California, United States. ffhal-00918200f" which corresponds to the preamble of claim 1.

In such case, the intervention is simulated through virtual probes, forming a cooling "Iceball" in their neighborhood, that is a predetermined cooling volume, for highlighting a further front of advancement of itself.

This solution is not efficient, as the so-called "Iceball" is simply a small cooling ball in the neighborhood of the needle in its insertion point. Such volume is generated without considering the essential parameters for having a maximum efficiency of cellular death, for example temperature and time to reach such temperature, so as that the risk of such "Iceball" causing the death of tumorous cells in lower amount than expected can occur.

Document US2009171203 A1 describes methods and systems for inserting therapeutic probes into a patient are present. Examples include an automated probe insertion system and a probe insertion guide comprising a moveable probe guide sleeve; a positioner for positioning said probe guide sleeve; and a controller operable to receive a definition of a locus within a body of a patient and to direct positioning and orienting of said probe guide sleeve so that a probe advanced through said sleeve will advance in direction of said locus. Cryosurgery applications are presented. A three dimensional model is useable by a simulator for simulating the cryosurgical intervention. The simulator comprises a displayer for displaying views of the model, and an interface useable by an operator for specifying loci for insertion of simulated cryoprobes and operational parameters for operation of simulated cryoprobes for cryoablating tissues. Thus, an operator (i.e., a user) can use the simulator to simulate a cryoablation intervention, by using the interface to command particular views of model, and by specifying both where to insert simulated cryoprobes into an organ imaged by the model, and how to operate the cryoprobes. Typically, an operator may specify positions for a plurality of the simulated cryoprobes, and further specify operating temperatures and durations of cooling for the cryoprobes. The display is then useable for displaying in a common virtual space an integrated image comprising a display of the three dimensional model a virtual display of the simulated cryoprobes inserted at said operator-specified loci,

Document US2006155267 A1 describes systems and methods for rendering visible to a cryosurgeon an estimated border of a cryoablation volume, thereby facilitating accurately delimited cryoablation of pathological tissues.

More particularly, the present disclosure relates to systems and methods for reading temperature and location information from images provided by imaging modalities and optionally from thermal sensors, inferring from that information the three-dimensional shape and position of a volume of tissue considered reliably cryoablated, and presenting this shape and position information to a cryosurgeon by integrating a visual model of that information with one or more images derived from standard imaging modalities, and displaying for a cryosurgeon that integrated image.

Disclosure of the invention

It is therefore the aim of the present invention to provide an innovative machinery for surgical interventions of cryoablative type, which solves said technical inconveniences.

In particular, it is the aim of the present invention to provide a machinery able to help the surgeon before and after the operation, so as to optimize the number, the insertion and the positioning of the probes in order to undermine more efficaciously ill tissues and preserve adjacent healthy tissues.

These and other aims are thus obtained with a machinery for surgical interventions of cryothermic type, as per claim 2.

Such machinery is equipped with a section (10) of virtual simulation as per claim 1.

In this way, all said technical inconveniences are easily solved.

In particular, it is now possible to simulate virtually an intervention of a 3D image which shows really the organ to be treated, by verifying visually the advancement of the cold front.

In this way, there is the certainty that the cold is really attacking the ill part and at the same time, fully preserving healthy tissues.

Such simulation will allow the surgeon to test also more than once the specific intervention and to find consequently the best solution regarding positioning, insertions and number of cryodes (both warm and cold) to be used.

Advantageously, the simulation processor is programmed for calculating such advancement of the cold front through partial differential equations.

Advantageously, the machinery is programmable according to any type of cryogenic protocol and to the capacity to receive the parameter of execution generated by the simulation section.

Further advantages are inferable form the remaining dependent claims.

Brief description of drawings

Further features and advantages of the present machinery, according to the invention, will result to be clearer with the description that follows of some embodiments, made to illustrate but not to limit, with reference to the attached drawings, wherein:

- Figure 1 shows in outline the acquisition of images with a system of medical diagnostics such as MRI scan or CAT, well known over the prior art;
- Figure 1A shows the processing of such images for obtaining a unique 3D image;
- Figure 2 shows the transfer of said 3D image to the simulation section 10, being part of the machinery object of the invention; the acquisition of diagnostic data can also be offline through digital filing supports or through the informative system of the structure;

- Figure 3 outlines a tridimensional image concerning a heart, obtained by data of medical diagnostics machineries;
- Figure 4A outlines a probe and its generally egg-shaped form generated by it;

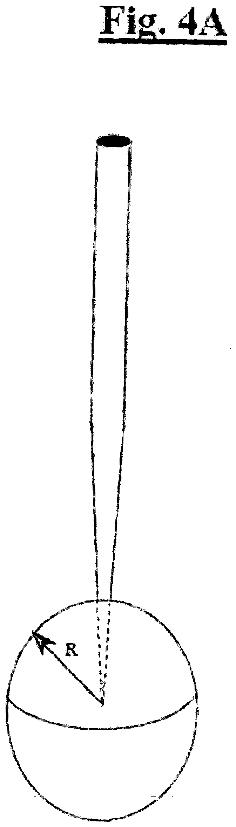

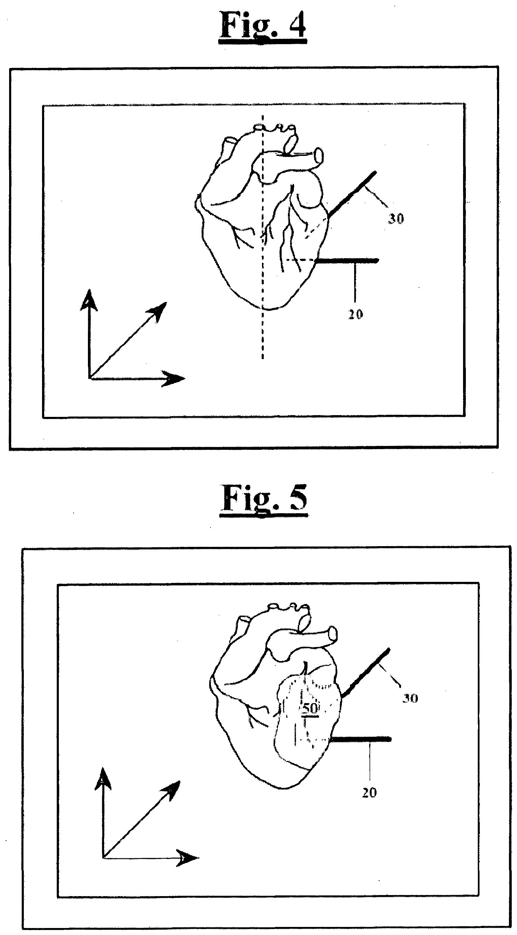

- Figures 4 and 5 outline a simulation of insertion of probes into the heart and its frozen area 50;

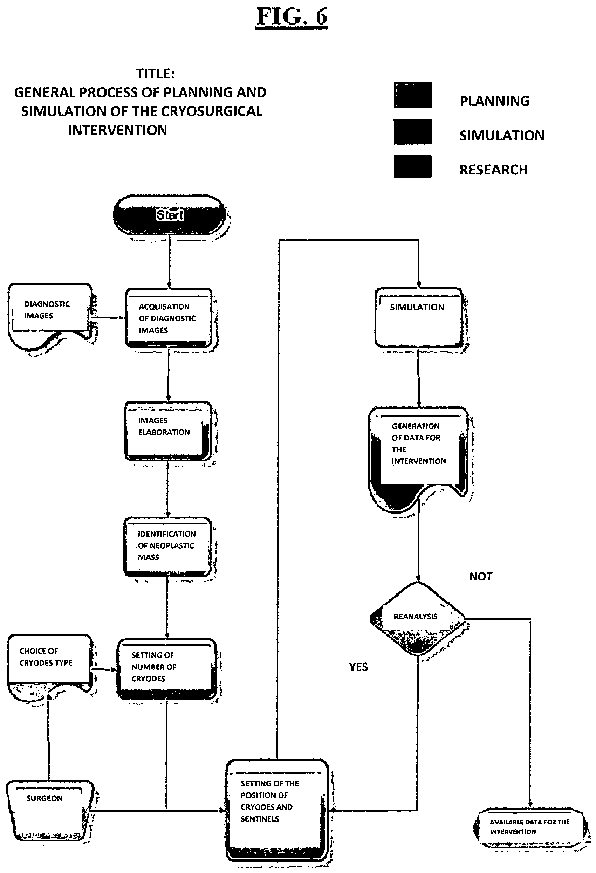

- Figure 6 is a flow chart which resumes the working phases of the machinery concerning the section of simulation;

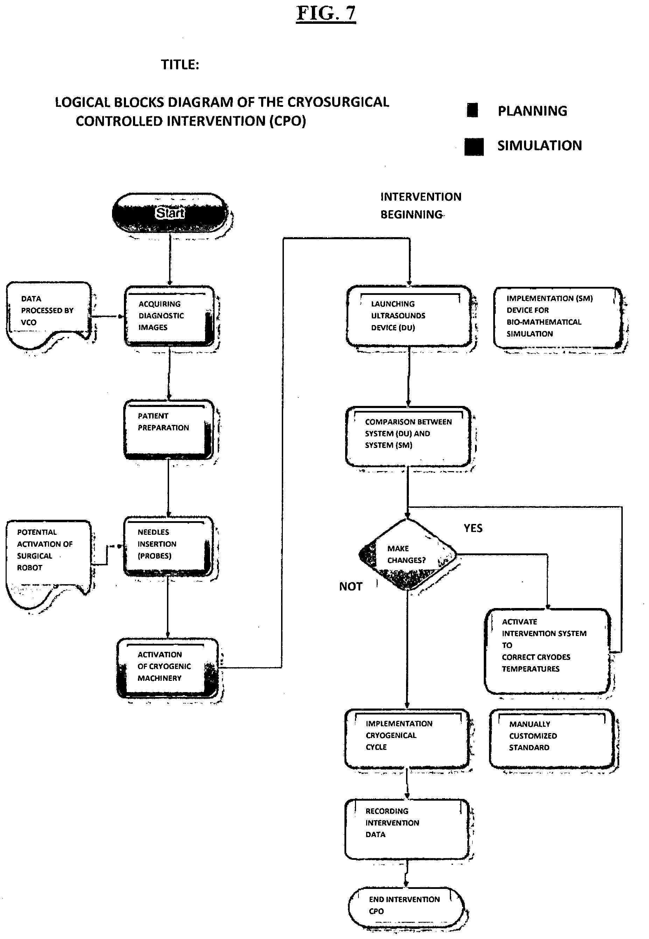

- Figure 7 is a flow chart concerning the phase of the proper surgical intervention;

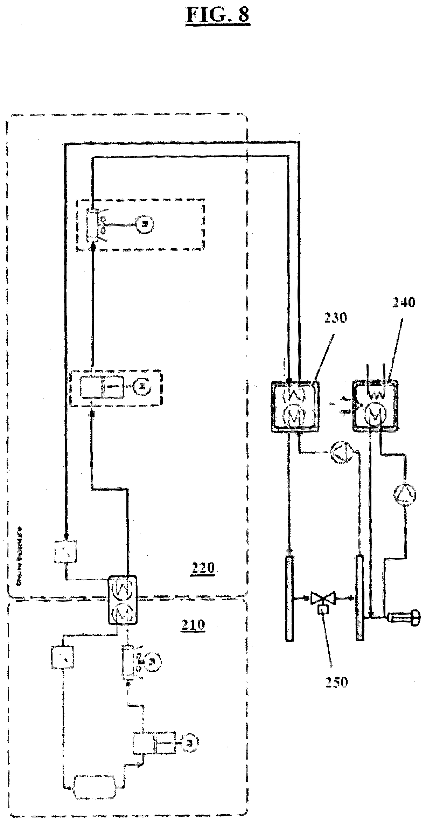

- Figure 8 is a diagram relative to the part deputed to the generation of the cold;

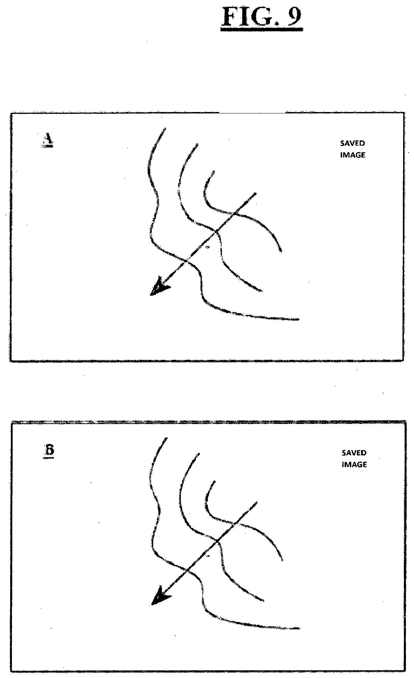

- Figure 9 is a comparison between a simulated image and an image acquired with ultrasounds during the proper intervention, so as to compare them and check potential differences what has been simulated and what is really occurring.

Description of some preferred embodiments

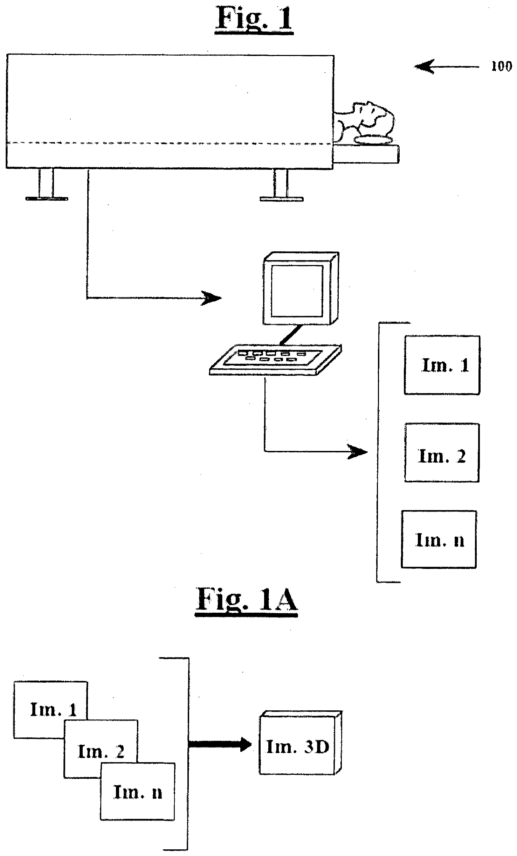

Figure 1 shows a phase of images acquisition of a hypothetical part to be treated.

The part to be treated can be an internal organ as well as an external part of the body, for example a portion of skin.

The image acquisition, above all in the medical field, is known and commonly used and acquisition systems, such as MRI scan or CAT, can be enumerated.

Therefore, figure 1 outlines a phase of acquisition of one or more images (Im.1...Im.n) through a traditional machinery 100 for MRI scan. The acquired images, in electronic format, can obviously be visualized and saved on a standard PC and, as better described further, are further elaborated.

Any method of images acquisition is not limited to what described in figure 1. For example, the images related to external parts (for example a mole) can be acquired through digital devices, such as digital cameras.

The acquired images are in electronic form and can obviously be sent or provided to any processing device.

It is therefore foreseen such elaboration phase of images, which can take place in a processor being part of the machinery object of the present invention, as well as in a separate processor, to be subsequently transferred to the machinery.

Specifically, such images, as outlined in figure 1A, are combined between them, in order to obtain a unique 3D image.

Modeling software of diagnostic systems images are sold and therefore they are not specific object of the present invention but here are suitably used. In general, once acquired an image, or rather a set of two-dimensional images from diagnostic systems such as CAT scan or x-ray, the processing system generates a 3D model from said images (therefore a single volume).

The received information has dimensional step (measurement scale), distance between the images and origin of the axes, that is, what is necessary to create compacting in a single (biological) solid, by combining the individual images. Dedicated software that structure the described solid in an appropriate grid exist. Basing on the colors or different brightness, the software also identifies neoplasia (or neoplasms) and highlights them with color (also by extrapolating them from the structure itself). Once identified the volumes, they will be "defined" with the same procedure by an appropriate grid that represents a three-dimensional matrix.

As better described further, said grid will be a support for mathematical applications of finite-state calculations of partial differential equations.

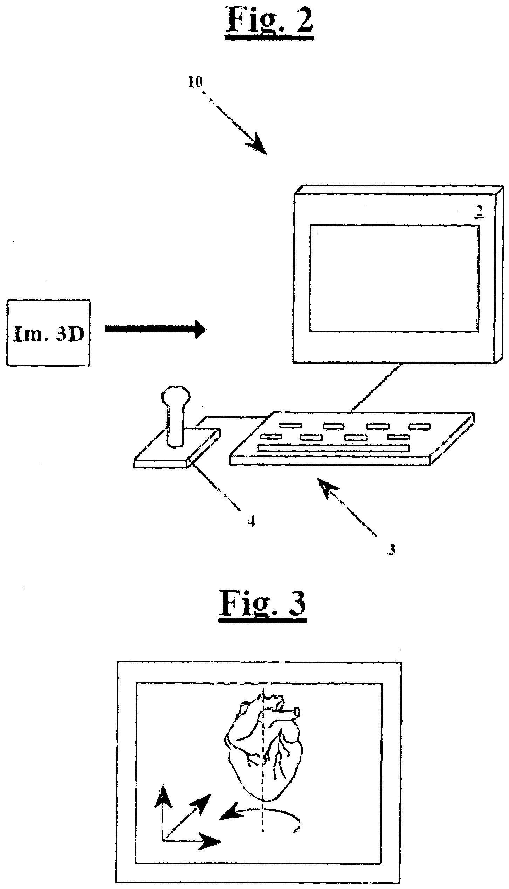

Figure 2, with number 10, outlines a part of such machinery and in particular the simulation section of the intervention.

One or more screens 2 and a specific processor 3 are foreseen for that purpose.

The processor, in a first embodiment of the invention, receives and saves the images which were beforehand obtained for example through a traditional system of MRI scan. The processor elaborates the images (see figure 1A) as described above, in such a manner that a unique tridimensional volumetric image is obtained, wherein the neoplastic mass is highlightable.

As alternative, as already said, the elaborated image obtained by starting from previous images of the MRI scan, x-ray or other ones, can be elaborated in a unique 3D image, not necessarily in the processor 3 of the machinery but in a separate processor. Therefore, the obtained final image can be sent or loaded to section 10 of figure 2. Files related both to acquired images of diagnostic devices and to created tridimensional models can be acquired by the processor 3 in various ways and also offline through digital filing supports or through the informative system of the structure.

In all cases, the processor 3 saves the tridimensional image, also called 3D model, depicting the organ or part of it and the user can control and move (for example rotate) the image related to the organ which appears on a specific video 2, for example through the mouse 4 or specific keys of the keyboard. The result of the rotated image will be visible on the screen in this way.

The handling of 3D models, such as rotation of tridimensional images, is a well-known software technique over the prior art and it is not a specific object of the present invention.

Therefore, 3D model can be rotated in different positions as needed by the user which simulates the intervention.

For example, figure 3 outlines an image depicting a heart in video 2 and this image can be freely rotated in different positions by the user.

In this way, the user can identify any part of the image.

Moreover, through well-known software devices, the user has the possibility to identify, delimit and mark (therefore segment) specific parts of tissue which prove to be ill according to the user's opinion, possibly in addition to those ones already highlighted by the 3D elaboration software.

The video representation of ill tissues, as already outlined, automatically identified and/or through the segmentation made by the operator, can be particularized through the use of different colors, as needed by the operator.

At this point, the simulation section allows to make the following two steps of real simulation, that is setting the number of necessary cryodes for attacking efficaciously the whole neoplastic volume identified in the specific image and then to simulate the insertion of such cryodes, by simulating the related advancement of the cold front.

In particular, according to the present invention, it is found that a volume of maximum efficacy exists or, anyway, it is conceivable, that is the area of greater cellular death. This volume is measured on characteristics of the thermal cycle to be applied according to Prof. Chua's theory of the maximum cellular death and to thermal-physical characteristics of the used probes.

Known the geometry of the probe, known its sizes and the energy transmitted to it, an equation is implementable determining a cooled volume (generally of egg-shaped form) and wherein an "efficient" cooling takes place, that is it guarantees almost the whole cellular death which represents actually a specific range of action of the probe.

More in details, the cooled effective volume is where almost the whole cellular death takes place and it is not the volume cooled below a predetermined temperature (generally below -30° Celsius degrees) but also and above all the cooling time, that is the time interval within which such temperature is reached (therefore, temperature and time needed to reach such temperature).

A preliminary calculation based basically on equations taking into account the geometry of the probe, the energy transmitted to it, in combination with the characteristics of the thermal cycle related to the theory of the maximum cellular death, allow to calculate such volume of maximum efficacy and thus to determine consequently the number of cryodes necessary to operate the whole volume of the ill area.

Therefore, known the type of cryodes available in the machinery, it is already programmed so as to know, for each cryode, which is the volume of maximum efficacy that it generates.

The function of calculation is of the type:

f (Shape and size of the cryode, energy transmitted to the cryode, minimum reached temperature, period for obtaining said temperature, tridimensional simulation of the biothermal advancement in respect of Prof. Chua's rules or of the required cryosurgical procedure).

Therefore, figure 4A outlines a single cryode and the volume which represents the efficient one, coolable by it, according to the geometry of the cryode itself and the energy that is transferred to it.

This perspective is important as, in the initial phase of the simulation, at the moment when the surgeon inserts cryodes to the image, the image allows to visualize such initial action spaces of each cryode with different colors, so it gives to the surgeon an immediate idea of the size of the neoplasia on the base of the selected initial position. In fact, this one becomes a visual instrument which helps to determine the initial position of the cryodes.

After that, as explained better below, the system resets itself and the proper advancement of the cold front starts from body temperature.

Section 10 allows to proceed with the proper simulation, that is the insertion of cryodes and the simulation of advancement of the cold, referring to figures 4 and 5.

Knowing the advancement of the cold is essential, as, once inserted the necessary cryodes to cover the ill volume, nevertheless, as time passes by, the cold advances and undermines or can undermine healthy tissues. The advancement of the cryogenic front develops itself in the combination of cold fronts of all cryodes, by generating geometries which are considered at the moment during such simulation in the virtual intervention.

Starting from the positioning of necessary cryodes, the simulation which calculates the advancement of the cold front allows to check not only if the positioning and the insertion of cryodes is exact (therefore, if it undermines the whole ill area on the base of the initial positioning according to a maximum efficacy volume), but also how to contain the advancement of the cold front if it is necessary.

More in details, as outlined in figure 4, the processor allows to identify virtual probes (20, 30) (of which the overall necessary prior number is known, as said above) which can be positioned in various positions of the organ, depending on needs of the user, in order to cover the whole ill area through the initial visualization of the efficient volume produced by each single probe as said above.

Therefore, the user rotates the image into the more suitable position for him and time after time he selects a probe and inserts it through an appropriate hole and to an appropriate depth inside the organ.

After having inserted all probes for covering the ill part, a proper simulation phase begins actually for checking the advancement of the cold front.

Once the insertion is completed, the user proceeds with a consensus to the processor 3 which calculates, depending on number and position of the probes, the advancement of the cold front, by highlighting it in the image itself.

The advancement of the cold front can also be visualized gradually on the screen with isotherms of different colors or coloring gradually the area hit by the cold front.

This part is essential, as, actually, the cold spreads in the tissue in such a manner that it could undermine the healthy tissue.

Its advancement depends on the structure of the tissue and on its characteristics and therefore its prevision is based on a mathematical rule different from those one used for calculating the number of probes.

In the case of the advancement of the cold front, partial differential equations, as explained further, are used, while, in the case of calculating a number of necessary probes (and thus of efficacy volume), the characteristics of the probe and the transmitted energy are mainly taken into consideration, as already said above.

As said, the development of the cold front is calculated on the base of a mathematical theory through the use of complex but well-known partial differential equations. The mathematical system solves the partial differential equations related to the advancement of the low temperature by using finite state systems, point by point, calculating thermal values in respect of the preset conditions to the neighborhood.

Such equations are an exact mathematical model which allows to check how the cold advances inside a preset body volume.

Other parameters have to be known, such as the position of insertion of the probe/s (that is its surface in contact with the organ), the nominal performances, such as reachable temperature and the morphology of the probe.

Considering a single probe, it determines an advancement zone of cold (generally almost spherical shaped). More cold probes determine the intersection of such volumes thus determining more complex geometries that can be highlighted with a specific mathematical model based, as said, on partial differential equations. In order to contain the action of the cold and limit precise areas, cold probes are cooperating with one or more warm probes, whose temperature is maintained between 38°C and 40°C Celsius degrees (therefore close or similar to the human body).

The aim of these specific probes is the protection of healthy tissues/organs near the neoplastic mass, object of the cryoablative intervention. Instead, the cold probe reaches temperatures of approximately -130°C.

Therefore, both warm and cold probes are inserted in the virtual simulation, depending on the operator's discretion and the processor 3, through said partial differential equations, highlights the geometry of the area where to insert the probes and determines the actual cooled volume.

Such cooled volume can (for example in the image) be visualized through a color gradient for recognizing the various isothermal curves. An example is shown in figure 5 where number 50 highlights a blackened area (which indeed represents the frozen area).

In this way, visually, it can be immediately identified if the insertion and the initial positioning of the probes is optimal (and therefore if it covers efficaciously the whole neoplasia) or if the insertion and the selected positioning occupies excessively healthy tissue or leaves ill tissue untouched.

Therefore, the surgeon can simulate in this way various positioning on the same image, also by varying the number of initial cryodes, until he determines the ideal positioning, identified not only by the number of used probes but also by their positioning and depth of insertion.

From a strictly mathematical point of view, used partial differential equations foresee the use of Stefan's equation, which deals with the heat transmission into biological tissues (also known as Stefan's direct problem). Taking into account that a cold front is transmitted, the thermal count of blood and of potentially close organs (that obstructs the progression of the cryogenic effect) is considered in equations. From those equations, then Pennes equation is considered, being essential for our purposes. After suitable thermal-mathematical changes, we arrive at the modified Pennes equation, which is applied in numerical field. The process of heat transfer is described in terms of the correspondent temperature field (x; t), wherein x is the position vector and t is time. Partial differential equations for the temperature field in areas V1, V2 and V3 follow the preceding assumptions, as the related border and initial conditions. In the unfrozen region, V3, the temperature satisfies the classical heat balance equation for biological tissues.

In the frozen region, V1, the equation simplifies in:

$$\rho_1 c_1 \frac{\partial \theta}{\partial t} = \nabla \cdot (k_1 \nabla \theta). \qquad (2.9)$$

$$\rho_3 c_3 \frac{\partial \theta}{\partial t} = \nabla \cdot (k_3 \nabla \theta) + \omega_b \rho_b c_b (\theta_b - \theta) + q_m. \qquad (2.8)$$

The mathematical model, in addition to the efficient volume of the probe and the further simulation of the cold advancement, allows also optionally to suggest a minimum number of necessary probes.

In this case, the equation of thermodynamics is used:

$$\Delta Q \quad = \quad Cs \quad \cdot \quad m \quad \cdot \quad \Delta T$$

Wherein:

$\Delta Q$ is the calculated heat, $Cs$ is the specific heat, $m$ is the mass to be cooled, $\Delta T$ is the thermal interval. In this way, once calculated the necessary heat amount for cooling the mass, we can trace out the suggested number of cryodes.

Such simulation data are then saved from the processor of the machinery.

In particular, the machinery, having calculated an image as a function of an initial reference, is able to save the coordinates of each probe in respect to the organ outlined in the image, by knowing positioning and depth.

The data memorization is relevant as it allows to help the surgeon with the following proper intervention.

The flowchart of figure 6 describes very well the phases described until now.

In particular, acquired diagnostic images are sent to the simulation section which processes such images and allows to identify the neoplastic mass. Therefore, the operator has the opportunity to select probes and sentinels (warm probes) for positioning them as needed and for proceeding with a new simulation.

Saved data comprise also particular markers related to main moments of temperature distribution, that is data related to images of advancement of the cold front are saved during the insertion and the permanence of cryodes.

Such markers, named logical Key performance indicator KPI, are made available on the screen during the intervention, in order to be used as reference term on the process itself. In particular, the processor compares the real advancement of the cold front to the saved reference one and can generate an alert, for example a sound alarm, if the cold course is corresponding basically to the simulated one.

More particularly, the simulation of the advancement cryogenic front displays with isothermal or isocryothermal lines evolving over time. All effects of different cryodes combine inside the volume thus forming curves that represent the "advancement of the cold front inside the tissue". They can be compared to isoaltimetrical lines of elevations. During the cooling, these curves evolve, that is they distance themselves from the origin of cryodes. It is a path developing over time. From the time zero (beginning of the cooling phase), they move gradually from the probe. Initially, they are small almost spherical volumes that, after suitable times (function of the topological-biological characteristics) melt together thus creating the above-mentioned iso-cryogenical lines. At this point, it is clear that, from the simulation phase, it can be known the position taken by such curves. Therefore, a frame of such iso-cryogenical path can be saved at all times, as a function of inserted probes. The same one takes place by using ultrasounds. Ultrasounds allow to check the cold advancement on the real organ and therefore the images related to the organ shot by ultrasounds are compared to frames. The further ones will represent graphically images representing the evolving of the cold front. The comparison takes place by checking if there are the same temperatures in a certain position (space) and at a certain moment (time).

Figure 9 outlines a comparison (Im A and Im B) between the simulated cold advancement and saved at a predetermined moment (**T1**) (therefore an image A of the simulation) and what detected during the intervention and saved during the intervention with ultrasounds at the same time (**T1**) (therefore an image B saved through ultrasounds at the same moment during the intervention). The example shows a good compliance between A and B, thus indicating a real trend similar to the simulated one.

This is a further instrument which allows to check if what was simulated is consistent with what is taking place during the intervention, possibly allowing a certain margin of correction at the surgeon's discretion.

Different simulations of the same intervention can be saved in such a manner that terms of comparison and analysis of the various cryogenical activities can be reported. It can be realized through a specific instrument for analysis and comparison which allows to visualize at the same time the main data of more recorded simulations.

At this point, once saved the data, one can proceed with the proper intervention.

For that purpose, the flowchart of figure 7 outlines the essential phases.

The data processed in the previous phase are made available for the section of the machinery which manages the operation and the optional external system for the guided positioning of the probes (for example a robot).

The system generates data of positioning that can be made available for systems for the guided positioning of probes (IGT Image Guided Therapy), very well-known over the prior art.

Therefore, needles are inserted respecting previously saved coordinates.

The robot is an unessential option for the present patent. There are in fact operative cases in common surgical procedure that are specific of the positioning within human organs.

An important feature of the machinery concerns also, in its complex, the used cooling system.

In particular, over the prior art, liquid nitrogen contained in tanks is used and inserted through a simple open circuit. The supply of liquid nitrogen is expensive and its treatment is dangerous.

In theory, cooling system with closed cycle have been proposed, exactly like refrigerating circuits wherein a fluid of thermal exchange is made circulate, for example Freon which generates cold through compression and expansion cycles. In that sense, the advantage of such systems is that no more supply of liquid nitrogen is necessary and, in addition, it is enough to connect the system to a socket to make it work normally like a domestic refrigerator.

It is also possible (and preferred) that said machinery is previously "charged" that is prepared in all its cryogenical parts outside the operating theatre, in order to operate without any need of electricity. Some stopgap batteries can be foreseen for the operation of electronical devices. Regarding this matter, it is to be reminded that a cryosurgical intervention does not take much time.

Nevertheless, it is necessary to reach particularly low temperatures, as already said around -130°C, and for this reason, gasses used in the normal cooling technique (for example Freon) are not enough.

It has been proposed a solution based on cooling fluids, such as, only to mention a few, octafluoropropane or perfluoropropane which is named super-greenhouse gas, that is one thousand time more dangerous than $CO_2$. However, such gasses are dangerous for human health and, as already said, have strong environmental impact. Therefore, in case of hole in the duct of the circuit, there is a serious risk of death by poisoning of all subjects, that is patient and health workers.

Therefore, the solution of the invention foresees the use of a so-called R14 fluid (tetrafluoromethane, formula:

$CF_4$) which will have the effect of generating temperatures around -120°C, once liquefied around -45°C.

Such fluid is made expand through an expansion valve or a suitable hairline pipe, thus reaching easily said temperatures.

This fluid has a high stability of the C-F bond which means that the compound does not destroy the ozone layer. Said cooling system is named **closed cycle,** because it requires no external cooling power supply.

Following figure 8 shows a scheme representing the cooling system of the machinery.

The figure shows a primary circuit 210 and a secondary circuit 220. Then there is a managing section of fluid 230 and a warm section 240 with electrical heating. In figure 8, headline pipes (or lamination valve) of the two (primary and secondary) circuits are not visible but they can be thought correctly as operating inside the primary ones of exchangers.

Two cooling fluids operate inside it, one of them is for reaching the minimum temperature around -40°C. Its function is to liquefy the second fluid F14, by using a heat exchanger. This further one will decrease the temperature inside the tank around -130°C, by expanding in the suitable duct.

It is also foreseen a third liquid (inside the tank) which, cooled around -100°C, will transport the cooling energy to the probes. We suggest alcohol, consisting basically of ethyl alcohol with the addition of safety components.

Such liquid, pumped at low speed and pressure, will go to a manifold with rapid clutches for the insertion of different probes (cryodes) which will be subjected to thermal decrease.

In addition to the two (cryogenical, primary and secondary) sections, there is a third one, whose function is to maintain a warm fluid, similar to the above-mentioned one, in order to heat sentinel needles (warm section).

In addition, we highlight that Prof. Chua's cycle recommends a decrease and also a return to body temperature repeated several times. The system exposes its cycle based on required medical protocols, that is an operating protocol which is based on Chua's protocol (Chua KJ, Chou SK, Ho JC. An analytical study on the thermal effect of cryosurgery on selective cell destruction. Journal of Biomechanics 2007; 40: 100 - 116).

There is also the possibility to propose to the user the insertion of a preferred cycle and therefore to change the specific protocol as needed, depending on requirements.

The present "thermal vector" system allows to use probes of different sizes (useful for various types of cryosurgical intervention) and the insertion related to the cryo-balloon technique for atrial fibrillation interventions (A-Fib). The probes insertion will be facilitated by rapid and safe connections (and also by low pressure of the vector fluid).

The aim of the "warm" function is to realize easily this cycle. There must be the possibility to interrupt easily the intervention (by heating cryodes rapidly indeed).

That is heating as rapidly as possible the probes in order to extract them. This operation is named "rapid release of cryodes". It is therefore obvious the function and the usefulness of the warm section.

## Claims

1. A virtual simulation section (10) for simulating an intervention of cryoablation, said section comprising:

   - A video device (2) for visualizing at least a three-dimensional image (Im) of an organ or part of the body on which the intervention has to be performed;
   - A processor (2, 3, 4) programmed for generating one or more virtual probes (20, 30) for causing a decrease of the temperature in their neighborhood and for calculating an advancement of the cold front (50) in the image (Im) depending on the positions wherein said probes are prearranged (20, 30);

   **Characterized in that:**

   - Said processor (2, 3, 4) is further programmed for calculating a volume of maximum efficacy of one or more predetermined probes (20, 30), said volume of maximum efficacy being a volume wherein a predetermined temperature (T) is reached within a predetermined time interval (t) and being determined through a function (f) depending at least on the geometry of the probe and on the energy transmitted to it and wherein said processor is further programmed for determining a number of probes necessary for cooling according to the volume of maximum efficacy a predetermined volume to be treated;
   - Said processor being programmed for generating consequently said number of virtual probes (20, 30) and for calculating the advancement of the cold front (50) into the image (Im) in function of the positions wherein said probes are positioned, said cold front being implemented through a model of partial differential equations which takes into account at least the morphology of the probe and said number of the inserted probes.

2. A machinery for surgical interventions of cryoablation comprising a virtual simulation section (10) according to claim 1.

3. A machinery, as per claim 2, wherein said simulation processor is further programmed for allowing the movement of the image (Im) in the video device (2).

4. A machinery, as per claims 2 to 3, wherein a section is provided and programmed for recreating a unique tridimensional image (Im) starting from one or more previously acquired bi-dimensional images.

5. A machinery, as per claim 4 wherein said section for recreating a unique tridimensional image starting from one or more acquired bi-dimensional images is the processor (2, 3, 4) being part of the simulation section (10) or a separate processor.

6. A machinery, as per claims 2 to 5, wherein said virtual simulation section (10) is configured for memorizing the positions of the probes and/or images related to the advancement of the cold front.

7. A machinery, as per claims 2 to 6, wherein the interfacing with an apparatus for generating ultrasounds is foreseen for the acquisition of related diagnostic images, and wherein a comparison is comprised between one or more saved images related to the simulation with one or more images detected with the connected ultrasounds apparatus, preferably being foreseen the generation of an alarm when the advancement of the cold front, identified through ultrasounds, moves away beyond a predetermined range from the simulated one.

8. A machinery, as per claims 2 to 7, wherein a system for generating cold is provided including a closed refrigerating system within a fluid R14 circulates.

9. A machinery, as per claims 2 to 8, wherein one or more stopgap batteries are provided in such a manner that the machinery can be previously charged thus resulting energetically independent during the intervention.

10. A machinery, as per claims 2 to 9, wherein said simulation section is separated from the section of the machinery related to the managing of the intervention.

11. A machinery, as per claims 2 to 10, wherein a section of the machinery is related to the managing of the intervention and preferably comprises an external system of guided positioning of the probes.

12. A machinery, as per claim 2, wherein the maximum efficacy volume is obtained with a function (f) depending on the shape and size of the cryode, on the energy transmitted to the cryode, on the minimum reached temperature and on the times for obtaining said temperature.

13. A machinery, as per claim 2, wherein said partial differential equations foresee the use of the Stefan equation and Pennes equation.

14. A method for doing a virtual simulation of an intervention of cryoablation, said method comprising the phases of:

- Arrangement of a video device (2) for visualizing at least a tridimensional image (Im) of an organ or part of the body to be undergone to the intervention and generation of said tridimensional image;
- Generation through a processor of one or more virtual probes (20, 30) for causing a decrease of the temperature in their neighborhood and for calculating an advancement of the cold front (50) in the image (Im) depending on the positions wherein said probes are prearranged (20, 30);
- And wherein the method comprises the following phases:

- Calculation, through said processor (2, 3, 4), of a volume of maximum efficacy of one or more predetermined probes (20, 30) said volume of maximum efficacy being a volume wherein a predetermined temperature (T) is reached within a predetermined time interval (t) and being determined through a function (f) depending at least on the geometry of the probe and on the energy transmitted to it.
- Calculation, with said processor, of a number of probes necessary for cooling a predetermined volume to be treated, according to the volume of maximum efficacy;
- Generation, with said processor, of said number of virtual probes (20, 30) above determined and calculation of the advancement of the cold front (50) in the image (Im) depending on the positions wherein said probes are prearranged, said advancement of the cold front being implemented through a model of partial differential equation which takes into account at least the morphology of the probe and said number of the inserted probes.

**Patentansprüche**

1. Ein virtueller Simulationsbereich (10) zum Simulieren eines Kryoablationseingriffs, wobei der genannte Bereich umfasst:

   - ein Videogerät (2) zur Visualisierung mindestens eines dreidimensionalen Bildes (Im) eines Organs oder Körperteils, an dem der Eingriff durchgeführt werden muss;
   - einen Prozessor (2, 3, 4), der so programmiert ist, dass er eine oder mehrere virtuelle Sonden (20, 30) erzeugt, um eine Verringerung der Temperatur in deren Nachbarschaft zu bewirken, und um ein Vorrücken der Kaltfront (50) in dem Bild (Im) in Abhängigkeit von den Positionen zu berechnen, in denen die Sonden (20, 30) angeordnet sind;

   **dadurch gekennzeichnet, dass:**

   - der genannte Prozessor (2, 3, 4) ferner programmiert ist, um ein Volumen maximaler Wirksamkeit einer oder mehrerer vorbestimmter Sonden (20, 30) zu berechnen, wobei das genannte Volumen maximaler Wirksamkeit ein Volumen ist, bei dem eine vorbestimmte Temperatur (T) innerhalb eines vorbestimmten Zeitintervalls (t) erreicht wird, und durch eine Funktion (f) bestimmt wird, die zumindest von der Geometrie der Sonde und von der auf sie übertragenen Energie abhängt, und wobei der genannte Prozessor ferner programmiert ist, um eine Anzahl von Sonden festzulegen, die zur Kühlung eines vorbestimmten zu behandelnden Volumens entsprechend dem Volumen maximaler Wirksamkeit erforderlich sind;
   - der genannte Prozessor ist programmiert, um folglich die genannte Anzahl von virtuellen Sonden (20, 30) zu erzeugen und um das Vorrücken der Kaltfront (50) in das Bild (Im) in Abhängigkeit von den Positionen, an denen die genannten Sonden positioniert sind, zu berechnen, wobei die genannte Kaltfront durch ein Modell von partiellen Differentialgleichungen implementiert wird, das zumindest die Morphologie der Sonde und die genannte Anzahl der eingeführten Sonden berücksichtigt.

2. Ein Vorrichtung für chirurgische Kryoablationseingriffe mit einem virtuellen Simulationsbereich(10) gemäß Anspruch 1.

3. Eine Vorrichtung gemäß Anspruch 2, bei der der Simulationsprozessor ferner so programmiert ist, dass er die Bewegung des Bildes (Im) im Videogerät (2) ermöglicht.

4. Eine Vorrichtung gemäß den Ansprüchen 2 bis 3, bei der ein Bereich vorgesehen und programmiert ist, um ein einzigartiges dreidimensionales Bild (Im) ausgehend von einem oder mehreren zuvor erfassten zweidimensionalen Bildern nachzubilden.

5. Eine Vorrichtung gemäß Anspruch 4, bei der der genannte Bereich zur Nachbildung eines einzigartigen dreidimensionalen Bildes ausgehend von einem oder mehreren erfassten zweidimensionalen Bildern der Prozessor (2, 3, 4) ist, der Teil des Simulationsbereiches (10) ist oder ein separater Prozessor ist.

6. Eine Vorrichtung gemäß den Ansprüchen 2 bis 5, bei der der virtuelle Simulationsbereich (10) so konfiguriert ist, dass er die Positionen der Sonden und/oder Bilder in Bezug auf das Vorrücken der Kaltfront abspeichert.

7. Eine Vorrichtung gemäß den Ansprüchen 2 bis 6, bei der die Kopplung mit einem Gerät zur Erzeugung von Ultraschall für die Erfassung zugehöriger diagnostischer Bilder vorgesehen ist, und der einen Vergleich zwischen einem oder mehreren gespeicherten Bildern, die sich auf die Simulation beziehen, mit einem oder mehreren Bildern, die mit dem angeschlossenen Ultraschallgerät erkannt werden, beinhaltet, wobei vorzugsweise die Erzeugung eines Alarms vorgesehen ist, wenn sich das Vorrücken der Kaltfront, die durch Ultraschall identifiziert wird, über einen vorbestimmten Bereich von dem simulierten Bereich hinaus bewegt.

8. Eine Vorrichtung gemäß den Ansprüchen 2 bis 7, bei der ein System zur Erzeugung von Kälte einschließlich eines geschlossenen Kühlsystems vorgesehen ist, in dem eine Flüssigkeit R14 zirkuliert.

9. Eine Vorrichtung gemäß den Ansprüchen 2 bis 8, bei der eine oder mehrere Notfallbatterien so vorgesehen sind, dass die Vorrichtung vorher aufgeladen werden kann, was zu einer energetischen Unabhängigkeit während des Eingriffs führt.

**10.** Eine Vorrichtung gemäß den Ansprüchen 2 bis 9, bei der der Simulationsbereich von dem Bereich der Vorrichtung getrennt ist, der sich auf die Durchführung des Eingriffs bezieht.

**11.** Eine Vorrichtung gemäß den Ansprüchen 2 bis 10, bei der ein Teil der Vorrichtung sich auf die Durchführung des Eingriffs bezieht und vorzugsweise ein externes System zur geführten Positionierung der Sonden umfasst.

**12.** Eine Vorrichtung gemäß Anspruch 2, bei der man das maximale Wirkungsvolumen mit einer Funktion (f) erhält, die von der Form und der Größe der Kryode, von der auf die Kryode übertragenen Energie, von der minimal erreichten Temperatur und von den Zeiten abhängt, um diese Temperatur zu erreichen.

**13.** Eine Vorrichtung gemäß Anspruch 2, bei der die partiellen Differentialgleichungen die Verwendung der Stefan-Gleichung und der Pennes-Gleichung vorsehen.

**14.** Ein Verfahren zur Durchführung einer virtuellen Simulation eines Kryoablationseingriffes, wobei das Verfahren die folgenden Phasen umfasst:

- die Anordnung eines Videogerätes (2) zur Visualisierung mindestens eines dreidimensionalen Bildes (Im) eines Organs oder Körperteils, das dem Eingriff und der Erzeugung des dreidimensionalen Bildes unterzogen werden soll;
- die Erzeugung einer oder mehrerer virtueller Sonden (20, 30) durch einen Prozessor, um in deren Nachbarschaft die Temperatur abzusenken und um eine Vorwärtsbewegung der Kaltfront (50) im Bild (Im) in Abhängigkeit von den Positionen zu berechnen, an denen die Sonden (20, 30) angeordnet sind;
- und wobei das Verfahren die folgenden Phasen umfasst:
- die Berechnung, eines Volumens maximaler Wirksamkeit einer oder mehrerer vorbestimmter Sonden (20, 30) durch den genannten Prozessor (2, 3, 4), wobei das genannte Volumen maximaler Wirksamkeit ein Volumen ist, in dem eine vorbestimmte Temperatur (T) innerhalb eines vorbestimmten Zeitintervalls (t) erreicht wird und das durch eine Funktion (f) bestimmt wird, die mindestens von der Geometrie der Sonde und der auf sie übertragenen Energie abhängt.
- die Berechnung einer Anzahl von Sonden mit dem genannten Prozessor, die zur Kühlung eines vorbestimmten zu behandelnden Volumens erforderlich sind, entsprechend dem Volumen der maximalen Wirksamkeit;
- die Erzeugung der oben festgelegten Anzahl virtueller Sonden (20, 30) mit dem genannten Prozessor und die Berechnung des Vorrückens der Kaltfront (50) im Bild (Im) in Abhängigkeit von den Positionen, an denen die Sonden vorher angeordnet worden sind, wobei das Vorrücken der Kaltfront durch ein Modell einer partiellen Differentialgleichung realisiert wird, das mindestens die Morphologie der Sonde und die genannte Anzahl der eingeführten Sonden berücksichtigt.

**Revendications**

**1.** Section de simulation virtuelle (10) pour simuler une intervention de cryoablation, ladite section comprenant :

- un dispositif vidéo (2) pour visualiser au moins une image tridimensionnelle (Im) d'un organe ou d'une partie du corps sur lesquels l'intervention doit être effectuée ;
- un processeur (2, 3, 4) programmé pour générer une ou plusieurs sondes virtuelles (20, 30) afin de provoquer une baisse de la température dans leur voisinage et de calculer une progression du front froid (50) dans l'image (Im) en fonction des positions dans lesquelles lesdites sondes sont pré-agencées (20, 30) ;

**caractérisé en ce que :**

- ledit processeur (2, 3, 4) est en outre programmé pour calculer un volume d'efficacité maximale d'une ou plusieurs sondes prédéterminées (20, 30), ledit volume d'efficacité maximale étant un volume dans lequel une température prédéterminée (T) est atteinte dans un intervalle de temps prédéterminé (t) et est définie par une fonction (f) selon au moins la géométrie de la sonde et l'énergie qui lui est transmise et dans lequel ledit processeur est en outre programmé pour déterminer un nombre de sondes nécessaires pour le refroidissement en fonction du volume d'efficacité maximale d'un volume prédéterminé à traiter ;
- ledit processeur étant programmé pour générer en conséquence ledit nombre de sondes virtuelles (20, 30) et pour calculer l'avancement du front froid (50) dans l'image (Im) en fonction des positions dans lesquelles lesdites sondes sont positionnées, ledit front froid étant mis en œuvre à travers un modèle d'équations diffé-

11

rentielles partielles qui prend en compte au moins la morphologie de la sonde et ledit nombre de sondes introduites.

2. Équipement pour des interventions chirurgicales de cryoablation comprenant une section de simulation virtuelle (10) selon la revendication 1.

3. Équipement selon la revendication 2, dans lequel ledit processeur de simulation est en outre programmé pour permettre le mouvement de l'image (Im) dans le dispositif vidéo (2).

4. Équipement selon la revendication 2 à 3, dans lequel une section est prévue et programmée pour recréer une image tridimensionnelle unique (Im) à partir d'une ou plusieurs images bidimensionnelles acquises précédemment.

5. Équipement selon la revendication 4, dans lequel ladite section destinée à recréer une image tridimensionnelle unique à partir d'une ou plusieurs images bidimensionnelles acquises est le processeur (2, 3, 4) faisant partie de la section de simulation (10) ou un processeur séparé.

6. Équipement selon la revendication 2 à 5, dans lequel ladite section de simulation virtuelle (10) est configurée pour mémoriser les positions des sondes et/ou des images liées à l'avancement du front froid.

7. Équipement selon la revendication 2 à 6, dans lequel l'interfaçage avec un appareil de génération d'ultrasons est prévu pour l'acquisition d'images de diagnostic associées, et dans lequel une comparaison est comprise entre une ou plusieurs images enregistrées liées à la simulation avec une ou plusieurs images détectées à l'aide de l'appareil à ultrasons connecté, en ayant de préférence prévu la génération d'une alarme lorsque l'avancement du front froid, identifié par ultrasons, s'éloigne au-delà d'une plage prédéterminée de celui simulé.

8. Équipement selon la revendication 2 à 7, dans lequel un système de génération du froid est fourni comprenant un système de réfrigération fermé dans lequel circule un fluide R14.

9. Équipement selon la revendication 2 à 8, dans lequel une ou plusieurs batteries provisoires sont prévues de telle manière que la machine puisse être préalablement chargée, ce qui la rend indépendante du point de vue énergétique pendant l'intervention.

10. Équipement selon la revendication 2 à 9, dans lequel ladite section de simulation est séparée de la section de l'équipement liée à la gestion de l'intervention.

11. Équipement selon la revendication 2 à 10, dans lequel une section de l'équipement est liée à la gestion de l'intervention et comprend de préférence un système externe de positionnement guidé des sondes.

12. Équipement selon la revendication 2,
dans lequel le volume d'efficacité maximal est obtenu avec une fonction (f) dépendant de la forme et de la taille de la cryode, de l'énergie transmise à la cryode, de la température minimale atteinte et des temps nécessaires pour obtenir ladite température.

13. Équipement selon la revendication 2,
dans lequel lesdites équations différentielles partielles prévoient l'utilisation de l'équation de Stefan et de l'équation de Pennes.

14. Procédé pour effectuer une simulation virtuelle d'une intervention de cryoablation, ledit procédé comprenant les étapes consistant à :

- agencer un dispositif vidéo (2) pour visualiser au moins une image tridimensionnelle (Im) d'un organe ou d'une partie du corps devant subir l'intervention et la génération de ladite image tridimensionnelle ;
- générer à l'aide d'un processeur une ou plusieurs sondes virtuelles (20, 30) afin de provoquer une baisse de la température dans leur voisinage et de calculer une progression du front froid (50) dans l'image (Im) en fonction des positions dans lesquelles lesdites sondes sont pré-agencées (20, 30) ;
- et où le procédé comprend les étapes suivantes, consistant à :
- calculer, à l'aide dudit processeur (2, 3, 4), un volume d'efficacité maximale d'une ou plusieurs sondes prédéterminées (20, 30) ledit volume d'efficacité maximale étant un volume dans lequel une température prédéterminée (T) est atteinte dans un intervalle de temps prédéterminé (t) et étant déterminé par une fonction (f) dépendant au moins de la géométrie de la sonde et de l'énergie qui lui est transmise.

- calculer, à l'aide dudit processeur, un nombre de sondes nécessaires pour refroidir un volume prédéterminé à traiter, en fonction du volume d'efficacité maximale ;
- générer, à l'aide dudit processeur, ledit nombre de sondes virtuelles (20, 30) déterminé ci-dessus et calculer l'avancement du front froid (50) dans l'image (Im) en fonction des positions dans lesquelles lesdites sondes sont agencées à l'avance, l'avancement dudit front froid étant mis en œuvre à travers un modèle d'équations différentielles partielles qui prend en compte au moins la morphologie de la sonde et ledit nombre de sondes introduites.

## Fig. 1

100

## Fig. 1A

## Fig. 2

## Fig. 3

# Fig. 4A

## Fig. 4

## Fig. 5

# FIG. 6

TITLE:
GENERAL PROCESS OF PLANNING AND
SIMULATION OF THE CRYOSURGICAL
INTERVENTION

PLANNING

SIMULATION

RESEARCH

Start

DIAGNOSTIC
IMAGES

ACQUISATION
OF DIAGNOSTIC
IMAGES

SIMULATION

IMAGES
ELABORATION

GENERATION
OF DATA FOR
THE
INTERVENTION

IDENTIFICATION
OF NEOPLASTIC
MASS

NOT

REANALYSIS

CHOICE OF
CRYODES TYPE

SETTING OF
NUMBER OF
CRYODES

YES

SURGEON

SETTING OF THE
POSITION OF
CRYODES AND
SENTINELS

AVAILABLE DATA FOR THE
INTERVENTION

# FIG. 7

TITLE:

**LOGICAL BLOCKS DIAGRAM OF THE CRYOSURGICAL CONTROLLED INTERVENTION (CPO)**

■ PLANNING

■ SIMULATION

INTERVENTION BEGINNING

Start

DATA PROCESSED BY VCO

ACQUIRING DIAGNOSTIC IMAGES

LAUNCHING ULTRASOUNDS DEVICE (DU)

IMPLEMENTATION (SM) DEVICE FOR BIO-MATHEMATICAL SIMULATION

PATIENT PREPARATION

COMPARISON BETWEEN SYSTEM (DU) AND SYSTEM (SM)

POTENTIAL ACTIVATION OF SURGICAL ROBOT

NEEDLES INSERTION (PROBES)

MAKE CHANGES?

YES

NOT

ACTIVATE INTERVENTION SYSTEM TO CORRECT CRYODES TEMPERATURES

ACTIVATION OF CRYOGENIC MACHINERY

IMPLEMENTATION CRYOGENICAL CYCLE

MANUALLY CUSTOMIZED STANDARD

RECORDING INTERVENTION DATA

END INTERVENTION CPO

# FIG. 8

# FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009171203 A1

- US 2006155267 A1

**Non-patent literature cited in the description**

- **HUGO TALBOT ; MYRIAM LEKKAL ; REMI BESSARD-DUPARC ; STEPHANE COTIN.** Interactive Planning of Cryotherapy Using Physically-Based Simulation. *MMVR 21 - Medicine Meets Virtual Reality - 2014,* February 2014

- **CHUA KJ ; CHOU SK ; HO JC.** An analytical study on the thermal effect of cryosurgery on selective cell destruction. *Journal of Biomechanics,* 2007, vol. 40, 100-116